# NOUVEAU FASCICULE DE BREVET EUROPEEN

(11) **EP 0 814 834 B2**
(45) Date de publication et mention de la décision concernant l'opposition: **18.03.2009**
(45) Mention de la délivrance du brevet: 13.09.2000
(21) Numéro de dépôt: 96906795.8
(22) Date de dépôt: 07.03.1996
(51) Int. Cl.: A61K 39/21, C07K 16/10, A61K 39/42, A61K 47/48, C12N 15/00

(54) **IMMUNOGENES DENUES DE TOXICITE DERIVANT D'UNE PROTEINE DE REGULATION RETROVIRALE, ANTICORPS DIRIGES CONTRE CES IMMUNOGENES, PROCEDE POUR LEUR PREPARATION ET COMPOSITIONS PHARMACEUTIQUES LES RENFERMANT**
VON EINEM RETROVIRALEN REGULATIONSPROTEIN ABSTAMMENDE, DETOXIFIERTE IMMUNOGENE, DAGEGEN GERICHTETE ANTIKÖRPER, VERFAHREN ZUR DEREN HERSTELLUNG UND DIESE IMMUNOGENE ODER ANTIKÖRPER ENTHALTENDE, PHARMAZEUTISCHE ZUSAMMENSETZUNGEN.
NON-TOXIC IMMUNOGENS DERIVED FROM A RETROVIRAL REGULATORY PROTEIN, ANTIBODIES DIRECTED AGAINST SAID IMMUNOGENS, PREPARATION METHOD THEREFOR, AND PHARMACEUTICAL COMPOSITIONS CONTAINING THE SAME

(30) Priorité: 08.03.1995 FR 9502708
(43) Date de publication de la demande: 07.01.1998
(73) Titulaire: NEOVACS, 75116 Paris (FR)
(72) Inventeur: ZAGURY, Jean-François, F-75003 Paris (FR); BIZZINI, Bernard, F-46100 Figeac (FR); ZAGURY, Daniel, F-75007 Paris (FR)
(74) Mandataire: Nargolwalla, Cyra
(86) Numéro de dépôt international: PCT/FR1996/000357
(87) Numéro de publication internationale: WO 1996/027389

(56) Documents cités:
- WO-A-89/12461
- WO-A-91/15224
- WO-A-91/18454
- WO-A-92/14755
- WO-A-93/22349
- WO-A-93/25235
- WO-A-94/15634
- WO-A-95/04546
- FR-A- 2 227 861
- FR-A- 2 700 169
- JOURNAL OF VIROLOGY, vol. 64, no. 2, BALTIMORE, pages 962-965, XP002006890 D. BRAKE ET AL.: "Characterization of murine monoclonal antibodies to the tat protein from human immunodeficiency virus type 1."
- THE JOURNAL OF CELL BIOLOGY D. BRAKE ET AL.: 'Identification of an Arg-Gly-Asp (RGD) cell adhesion site in human immunodeficiency virus type 1 transactivation protein, tat.' vol. 111, no. 3, NEW YORK, NY, ÉTATS UNIS, pages 1275 - 1281, XP002006891
- CELL, 55(6), 1988, 1189-93 XP008029605
- JOURNAL OF VIROLOGY, 68(5), 1994, 3343-53 XP000929493

## Description

La présente invention concerne de nouveaux immunogènes rétroviraux utilisant des protéines de régulation rétrovirales dont les propriétés biologiques essentielles auront été préalablement inactivées de façon telle que l'on conserve ou augmente leurs propriétés immunogènes, leur procédé de préparation et des compositions pharmaceutiques les renfermant. Ces nouveaux immunogènes "inactivés" peuvent être utilisés pour induire chez l'homme une immunisation active susceptible de prévenir ou corriger les effets de dérégulation que les protéines natives dont ils sont issus peuvent contribuer à produire.

De même, la présente invention concerne de nouveaux anticorps obtenus par l'utilisation de ces immunogènes "inactivés", leurs procédés de préparation et les compositions pharmaceutiques les contenant en vue d'une immunisation passive.

La molécule Tat est une protéine de régulation du VIH que l'on ne trouve pas dans la particule virale mais qui est codée par le génome du VIH-1. A l'intérieur des cellules infectées, cette protéine codée par l'ADN proviral joue un rôle transactivateur de gènes viraux ou cellulaires. Cette protéine de régulation génétique peut cependant se trouver également à l'extérieur des cellules dans le milieu circulant extracellulaire, excrétée au sein de débris de cellules mortes à l'état natif ou fragmentaire ou par processus de sécrétion. Sa présence dans le milieu circulant explique l'existence d'anticorps anti-Tat détectables chez certains sujets séropositifs. Dans ce contexte extracellulaire du Tat le segment C terminal porteur des résidus RGD reconnus par les intégrines de surface cellulaire et la région basique de la molécule (résidus 45 - 70) vont lui permettre, comme le font les toxines bactériennes, d'agir sur les cellules non infectées de différents tissus. Ainsi la protéine Tat circulante agissant comme une véritable toxine virale, peut exercer des effets nocifs sur les cellules endothéliales et en association avec le facteur de croissance BFGF, contribuer à la néoangiogenèse du Sarcome de Kaposi caractérisant la maladie Sida. La protéine Tat circulante peut également aggraver l'immunosuppression qui s'installe progressivement dans le Sida, soit par action immunosuppressive directe sur les cellules T soit en contribuant à dérégler la production d'interféron α par les cellules présentant d'antigène, appelées APC (macrophages ou cellules dendritiques).

Le syndrome d'immunodéficience acquise (Sida) est cliniquement défini par des maladies opportunistes dues à une immunosuppression ou par le Sarcome de Kaposi. L'immunosuppression acquise, d'installation progressive au cours de l'infection VIH, se manifeste biologiquement par la perte de réactivité immunologique des cellules T (cytostase et la diminution de production de l'IL2) après stimulation en premier lieu par les antigènes de rappel, puis par les alloantigènes et enfin par les mitogènes (PHA). Cette immunosuppression est associée à la surproduction des interférons α et γ.

Les mécanismes cytopathogènes qui induisent l'immunosuppression sont complexes et font intervenir différents facteurs d'origine virale agissant soit directement sur les cellules de l'immunité, lymphocytes T et APC, soit indirectement via le réseau des cytokines. Ainsi la protéine d'enveloppe gp120 portée par la particule de VIH-1 et dont la présence extracellulaire est mesurée par la charge virale sérique peut induire directement l'anergie de cellules T de phénotype CD4. De leur côté les protéines de régulation du VIH, notamment le Tat, dans sa configuration extracellulaire de toxine virale circulante, semblent également induire une immunosuppression directe des cellules T ou d'autres effets pathogènes, du fait par exemple qu'in vitro les cellules T activées par un antigène de rappel ou par des anticorps anti-CD3 ne prolifèrent plus en présence de la molécule Tat. En outre la protéine Tat circulante semble faciliter la surproduction par les APC d'interféron α, cytokine cytostatique et apoptogène susceptible d'amplifier l'immunosuppression et l'apoptose observés dans la maladie Sida. En effet la sécrétion d'interféron α par les macrophages ne semble plus, en présence de Tat, arrêtée par une rétrorégulation (feedback) qui à l'état normal contrôle cette production d'interféron α (période réfractaire).

Le Sarcome de Kaposi se manifeste cliniquement par l'apparition de nodules vasculaires représentant une néoangiogenèse constituée à partir de cellules endothéliales. Ces cellules activées par des processus inflammatoires engendrant la production de cytokines (interféron γ, IL1 et IL6) produisent du BFGF (Basic Fibroblastic Growth Factor) et se multiplient. La prolifération des cellules endothéliales activées in vitro est augmentée par la présence dans le milieu de la protéine Tat. Les anticorps anti-Tat bloquent in vitro les effets prolifératifs de la protéine Tat. L'action du Tat sur les cellules endothéliales porteuses à leur surface d'adhésine de la famille des Intégrines s'explique par la présence de la séquence RGD reconnue par ces molécules.

La néoangiogenèse qui sous-tend in vivo le sarcome de Kaposi serait donc favorisée par la protéine de régulation Tat dans sa configuration extracellulaire, qui pourrait être reconnue, grâce à son fragment C terminal contenant la séquence RGD, par les intégrines des cellules endothéliales. De plus le Tat pourra également agir par sa région basique riche en résidus K et R, et par conséquent susceptible de se lier à l'héparine sulfate de la matrice extracellulaire qui concentre le facteur de croissance BFGF. Ainsi la prolifération des cellules endothéliales induite par les facteurs de croissance est accrue par la présence de Tat et engendre la néoangiogenèse. Cet effet sur la croissance des cellules endothéliales est réduit in vitro par l'action d'anticorps anti-Tat.

Il apparaît donc souhaitable de bloquer l'activité nocive des protéines de régulation des rétrovirus, notamment du Tat circulant dans les milieux extracellulaires (sang, lymphe, milieux interstitiels ...), véritables toxines virales.

En ce qui concerne les virus VIH, on avait jusqu'à présent effectué des tentatives de vaccination à l'aide de protéines de structure ou fragments de protéines de structure de ces virus, mais jamais à l'aide de protéines de régulation ou fragments de protéines de régulation de ces virus.

Or, on a trouvé avec étonnement que, tout comme les toxines bactériennes (tétanique, diphtérique ou botulique) dont l'activité toxique est neutralisée par des anticorps spécifiques, les effets nocifs des protéines virales de régulation et notamment du Tat, véritable toxine du VIH dans sa configuration extracellulaire - qu'ils s'exercent par l'immunosuppression des cellules T, par le dérèglement de la production de l'interféron α par les APC ou par la néoangiogenèse qui sous-tend le Sarcome de Kaposi - sont abolis en présence d'anticorps spécifiques anti-Tat , comme on le verra ci-après dans la partie expérimentale.

Il en est de même avec d'autres protéines de régulation de virus tels que VIH-1, VIH-2, HTLV-1 ou HTLV-2. Il serait donc souhaitable de disposer d'immunogènes administrables à l'homme capables de produire de tels anticorps, de même que disposer de tels anticorps, à des fins tant curatives que préventives. En effet les composés de l'art antérieur utilisés comme immunogènes peuvent être toxiques pour l'homme (voir par exemple WO-A-9118454), notamment ceux comportant les régions basiques. Il s'agit de fragments non modifiés de Tat, de Nef ou de Rev "natifs", contrairement à la présente invention basée sur l'inactivation de ces protéines ou fragments de protéines.

WO-A-93 22349 décrit des anticorps naturels IgM de basse affinité qui sont présents naturellement dans le sérum d'individus non infectés par le VIH-1 et qui reconnaissent des épitopes sur des protamines, sur SP80 et sur Tat et suggère de les purifier à partir de sérums de sujets séronégatifs ou de surnageants de cellules B CD5+ clonées pour les utiliser en thérapeutique.

Le Journal of Virology, 1990, 64:962-5) décrit d'anticorps monoclonaux produits à partir de souris immunisées contre la protéine Tat en adjuvant de Freund. Certains de ces anticorps monoclonaux peuvent inhiber l'activité transactivatrice de la protéine Tat sur le LTR, promoteur de VIH-1.

Le Journal of Cell Biology, 1990, 111:1275-81 et WO-A-91/15224 décrivent la fixation de la protéine Tat peut sur des cellules monocytaires, lymphocytaires et musculaires grâce aux résidus RGD de sa séquence. Les récepteurs de Tat sur ces cellules peuvent être des intégrines. La demande PCT suggère d'utiliser des peptides ou des anticorps spécifiques des sites de liaison Tat-intégrine pour bloquer l'interaction RGD-intégrine et limiter l'effet extracellulaire de Tat.

En contradiction avec l'article et le brevet ci-dessus, WO-A-92 14755 identifie une région de Tat qui se lie aux intégrines et qui ne dépend pas de la séquence RGD, et propose d'utiliser en thérapeutique des inhibiteurs de la liaison tat-intégrine qui sont soit des fragments peptidiques provenant d'intégrines ou de Tat, soit des anticorps dirigés contre les sites de la liaison Tat-intégrine.

Cell, 1988, 55:1189-93 décrit la pénétration de la protéine Tat extracellulaire dans les cellules pour transactiver la transcription du promoteur du VIH-1 (CAT assay). Il décrit aussi la perte de cet effet transactivateur de transcription quand on utilise une molécule Tat traitée par carboxyméthylation.

Le Journal of Virology, 1994, 68:3343-53 décrit la production par synthèse de la protéine Tat native ou de fragments pour leurs propriétés transactivatrices sur le LTR du VIH-1, en utilisant comme marqueur le gène de la beta-galactosidase. Des formes modifiées de Tat par addition de groupements acétamidométhyl ont été aussi testées dans ce système de transactivation.

C'est pourquoi la présente demande a pour objet les compositions pharmaceutiques comprenant un composé immunogène administrable à l'homme car dénué de toxicité, caractérisées en ce que ledit composé immunogène dérive d'une protéine de régulation d'un virus VIH-1, VIH-2, ou d'un de ses fragments peptidiques par traitement à l'aide d'un agent chimique ou par couplage avec une protéine porteuse activée par un pré-traitement à l'aide d'un aldéhyde, lui permettant d'être reconnu par des anticorps dirigés contre ladite de protéine de régulation native, et de conserver des propriétés immunogènes suffisantes pour créer des anticorps neutralisant ou bloquant ladite protéine native, tout en ayant perdu au moins 50% des propriétés biologiques toxiques de ladite protéine native, ladite protéine de régulation étant la protéine Tat.

La présente demande a aussi pour objet les composés immunogènes tels que définis ci-dessus pour leur utilisation dans une méthode de traitement du corps humain ou animal, c'est-à-dire les médicaments caractérisés en ce qu'ils sont constitués des composés immunogènes tels que définis ci-dessus.

La présente demande a plus particulièrement pour objet des composés immunogènes tels que décrits dans les revendications ci-après.

Ces composés, par analogie aux toxoïdes bactériens tel que le tétanos toxoïde, seront qualifiés ci-après de "toxoïdes".

En effet, tout comme les toxoïdes bactériens classiques, ils sont dépourvus de toxicité propre, mais sont cependant capables de provoquer une immunisation par administration chez un sujet.

Le traitement chimique ci-dessous peut être complété par un traitement physique comme par exemple une irradiation et tout particulièrement une irradiation U.V., dans le but de diminuer la toxicité résiduelle des composés immunogènes selon l'invention.

Les toxoïdes ci-dessus peuvent par exemple être préparés à partir d'un peptide ayant une séquence identique ou similaire à une séquence peptidique d'une protéine de régulation, Tat, et être obtenu par exemple par synthèse peptidique conventionnelle sur résine ou par génie génétique. Tous ces procédés sont bien connus par l'état de la technique.

Afin de vérifier que la protéine de régulation modifiée ou son fragment modifié selon l'invention est bien reconnu par des anticorps dirigés contre ladite protéine de régulation native, on peut par exemple vérifier immunologiquement par Elisa en présence d'anticorps spécifiques, la formation de complexes antigènes-anticorps comme on le verra ci-après dans la partie expérimentale.

Afin de déterminer si les propriétés immunogènes de la protéine de régulation Tat ont été suffisamment conservées pour créer des anticorps neutralisant ladite protéine native, on peut par exemple immuniser des mammifères (lapins, rats, souris) à l'aide d'un composé immunogène selon l'invention et vérifier que les anticorps produits neutralisent les activités toxiques de la protéine de régulation, comme on le verra dans la partie expérimentale.

Afin de déterminer si la protéine de régulation Tat modifiée a perdu au moins de 50% de ses propriétés biologiques toxiques, on peut étudier l'effet de la protéine de régulation Tat inactivé sur l'immunosuppression des cellules T ou sur la production d'interféron α par des cellules mononucléées du sang périphérique activées ou encore sur la néoangiogénèse induite par la protéine de régulation.

L'inactivation de la protéine de régulation Tat est vérifiée par exemple par le " Tat Rescue Assay " utilisant un mutant de VIH non infectieux Tat déficient cultivé sur la lignée cellulaire HLM-1 dont la réplication dépend d'un apport exogène de Tat natif.

Le composé immunogène dérive de la protéine de régulation Tat des virus VIH-1, VIH-2.

On peut citer également tout particulièrement les régions extérieures aux régions basiques de Tat c'est à dire extérieures aux régions 49 à 57 de Tat ou chevauchant ces régions sur au plus 4 acides aminés, de préférence au plus 2 acides aminés, particulièrement le peptide HQVSLSKQPTSQPRGD.

Par « dérivent » ou « dériver » d'une protéine de régulation Tat d'un virus VIH1, VIH2, l'on entend que le composé immunogène peut être constitué de la totalité ou d'un fragment de la protéine de régulation et peut comporter une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés; les modifications doivent généralement concerner moins de 30% d'acides aminés, de préférence moins de 20% et tout particulièrement moins de 10% sur les segments d'homologie à la protéine de régulation d'au moins 8 acides aminés, notamment au moins 12 acides aminés. Un fragment peut comporter de 8 à 60 acides aminés par exemple, de préférence de 12 à 40 acides aminés, et particulièrement de 25 à 40 acides aminés. On peut citer par exemple les résidus 65-80 en C terminal du Tat de VIH-1.

Dans des conditions préférentielles, les composés immunogènes de l'invention comportent au moins 50% de la totalité de la protéine de régulation, de préférence au moins 70%, particulièrement au moins 90%, et tout particulièrement la totalité ou quasi-totalité de ladite protéine.

De manière générale, en ce qui concerne les modifications, l'homologie ou la similitude entre l'immunogène modifié et la protéine ou partie de protéine native, ainsi que les dimensions du composé immunogène, de même que les modalités d'utilisation, ou de couplage du composé immunogène selon l'invention à une protéine immunogène telle que le toxoïde tétanique, on peut en particulier se référer à WO-A-86/06 414 ou à EP-A-0.220.273 ou encore à PCT/US.86/00831, équivalents, dont l'enseignement est incorporé ici par référence.

Les composés immunogènes selon l'invention peuvent être utilisés comme suit :

On administre à un patient un composé immunogène selon la présente invention, par exemple par voie sous-cutanée ou intramusculaire, en quantité suffisante pour être efficace sur le plan thérapeutique, à un sujet ayant besoin d'un tel traitement. La dose administrée peut aller par exemple de 100 à 1000 µg par voie sous-cutanée, une fois par mois pendant trois mois, puis périodiquement en fonction du taux des anticorps sériques induits, par exemple tous les 2-6 mois.

Une composition selon l'invention peut être administrée par n'importe quelle voie conventionnelle en usage dans le domaine des vaccins, en particulier par voie sous-cutanée, par voie intramusculaire, par voie intraveineuse ou par voie orale. L'administration peut avoir lieu en dose unique ou répétée une ou plusieurs fois après un certain intervalle de temps.

C'est pourquoi la présente demande a également pour objet une composition pharmaceutique, curative ou préventive, caractérisée en ce qu'elle comprend à titre de principe actif, un composé immunogène tel que défini ci-dessus. Le composé immunogène peut être conditionné seul ou mélangé à un excipient pharmaceutiquement acceptable tel qu'un adjuvant.

L'invention a encore pour objet des médicaments caractérisés en ce qu'ils sont constitués des composés immunogènes tels que définis ci-dessus, à savoir des composés immunogènes ci-dessus pour leur utilisation dans une méthode de traitement thérapeutique du corps humain ou animal, ainsi que l'utilisation d'un tel composé immunogène pour la préparation d'un médicament curatif ou préventif destiné au traitement ou à la prévention des effets nocifs de la protéine de régulation Tat. En effet, les composés selon l'invention ont perdu leurs propriétés toxiques et peuvent donc être administrés chez l'homme comme on le verra ci-après dans la partie expérimentale.

L'administration de composés immunogènes selon l'invention correspond à une immunothérapie active. Il peut être intéressant également de procéder à une immunothérapie passive, c'est à dire de fournir à un patient directement des anticorps dont il a besoin pour neutraliser les effets nocifs de la protéine de régulation Tat des virus VIH-1, VIH-2.

Ces anticorps anti-protéine de régulation peuvent être obtenus classiquement et à titre d'exemple après immunisation d'un mammifère, homme ou animal, à l'aide d'un composé immunogène tel que défini ci-dessus, par clonage de lymphocytes B humains transformés par le virus Epstein-Barr puis récupération des anticorps attendus sécrétés par lesdits lymphocytes B transformés, ou encore par recombinaison génétique à partir d'une librairie de phages.

C'est pourquoi la présente demande décrit également de tels procédés de préparation d'anticorps anti-protéine de régulation d'un virus VIH-1, VIH-2 et en particulier d'anticorps anti-Tat toxoïde, et notamment un procédé de préparation d'un anticorps ci-dessus caractérisé en ce que l'on immunise un mammifère à l'aide d'un composé immunogène tel que défini ci-dessus.

La présente demande décrit encore un anticorps anti-protéine de régulation d'un virus VIH-1, VIH-2, et en particulier des anticorps polyclonaux ou monoclonaux obtenus à partir de sujets humains ou de mammifères immunisés par la mise en oeuvre des procédés ci-dessus décrits, à l'exception d'un anticorps anti-Tat.

Ces anticorps spécifiques pourront provenir :
1. soit du sujet lui-même, induits par une immunisation active (vaccination) avec une protéine de régulation biologiquement inactivée mais immunogène, notamment le Tat. Un tel composé immunogène, dans le cas par exemple du Tat est appelé Tat-toxoïde par analogie aux toxoïdes bactériens,
2. soit d'un organisme étranger, allo- ou xénogénique, administré au sujet par immunisation passive (sérothérapie). Les anticorps allogéniques (chez l'homme) pourront être engendrés chez des sujets non infectés volontaires après immunisation active (vaccination) avec une protéine immunogène ou ses dérivés selon l'invention, notamment du Tat (Tat-toxoïde ou fragments peptidiques du Tat selon l'invention). Ces anticorps administrés passivement, qu'ils soient allogéniques (humains) ou xénogéniques (animaux), pourront être des anticorps monoclonaux ou polyclonaux complets ou des fragments F(ab')2 ou Fab de l'anticorps.

Par « anticorps anti-protéine de régulation », l'on entend des anticorps monoclonaux ou polyclonaux ou des fragments F(ab')2 ou Fab de ces anticorps ou encore des anticorps anti-protéine de régulation obtenus par construction génétique à partir d'une librairie de phages.

Les anticorps allogéniques d'origine humaine sont :
- soit polyclonaux - pouvant être obtenus chez des sujets séronégatifs volontaires immunisés avec un composé immunogène selon l'invention, notamment du Tat-toxoïde ou des fragments peptidiques de Tat.
- soit monoclonaux à partir de clones spécifiques de cellules B transformées par le virus EBV de ces individus immunisés (lignées B EBV spécifiques).

Les anticorps xénogéniques proviennent d'animaux hyperimmunisés avec un composé immunogène selon l'invention, Tat ou ses dérivés (Tat-toxoïde, fragments peptidiques de Tat détoxifiés selon l'invention), et sont
- soit polyclonaux provenant d'animaux hyperimmunisés,
- soit monoclonaux, obtenus après hybridation selon la technique que Kohler et Milstein de cellules spléniques ou d'adénocytes avec une lignée myélomateuse, type x63, notamment x63AG3. Dans ce cas on préfère les anticorps équins ou de lapin.

La présente demande décrit encore un procédé de préparation d'anticorps anti-protéine de régulation d'un virus VIH-1, VIH-2, caractérisé en ce que l'on immunise un mammifère, homme ou animal, avec un composé immunogène tel que défini ci-dessus.

La présente invention décrit aussi un procédé de préparation d'anticorps monoclonaux anti-protéine de régulation d'un virus VIH-1, VIH-2, caractérisé en ce que l'on utilise des cellules B provenant d'individus immunisés par un composé immunogène selon la présente invention, lesdites cellules B étant transformées par le virus EBV et produisant des anticorps spécifiques anti-protéine de régulation d'un virus VIH-1, VIH-2.

Les cellules EBV + ci-dessus peuvent être cultivées de manière à produire les anticorps attendus. Ces cellules, comme on l'a vu, proviennent notamment de malades immunisés par une protéine de régulation native, ou par un composé immunogène selon l'invention.

La présente demande décrit un procédé de préparation d'anticorps selon l'invention monoclonaux, dirigés contre une protéine de régulation d'un virus VIH-1, VIH-2 inactivée ou non, caractérisé en ce que l'on prépare des hybridomes de mammifère, notamment de souris à partir de splénocytes ou adénocytes notamment de souris immunisées par une protéine de régulation native ou un composé immunogène selon l'invention, et de cellules de myélome, de préférence de lignée x63, selon les procédés bien connus de l'état de la technique (Kohler et Milstein).

La présente demande décrit un procédé d'obtention d'anticorps anti-protéine de régulation d'un virus VIH-1, VIH-2 par la technologie de recombinaison génétique, caractérisé en ce que l'on utilise à titre d'immunogène un composé immunogène tel que défini ci-dessus.

La présente demande décrit des fragments F(ab')2 ou Fab desdits anticorps ; ceux-ci peuvent être obtenus par digestion enzymatique par exemple.

La présente invention décrit un procédé d'immunisation passive de sujets contaminés par le virus VIH, utilisant des anticorps spécifiques anti-protéine de régulation de la multiplication du virus VIH et spécialement anti-Tat neutralisant ou bloquant les effets nocifs de cette protéine dans sa configuration extracellulaire et préparés comme indiqué ci-dessus, ou des fragments F(ab')2 ou F(ab) de ces anticorps.

La présente demande décrit un procédé d'immunisation active anti-VIH ou Sida utilisant un composé immunogène décrit ci-dessus, associé à une protéine constitutive (ou de structure) d'un virus VIH, notamment la glycoprotéine d'enveloppe gp 120 ou gp 160 ou un fragment peptidique modifié ou non de celles-ci, ou au virus VIH inactivé, ou au virus VIH deplété de son ARN génomique par exemple par hydrolyse alcaline.

La présente demande décrit un procédé d'immunisation active anti-VIH ou - Sida utilisant un composé immunogène décrit ci-dessus, associé à un ou plusieurs immunogènes à base de cytokines inactivées et spécialement l'interféron α, le TGFβ ou le TNF. En effet, comme on le verra ci-après dans la partie expérimentale, une combinaison des effets des anticorps selon l'invention contre des protéines de régulation et des anticorps anti-interféron α en particulier, restaure complètement l'immunosuppression induite par le VIH.

C'est pourquoi la présente demande a encore pour objet une composition renfermant deux composés immunogènes, à savoir un composé immunogène ci-dessus décrit, et un composé immunogène capable d'induire des anticorps contre une cytokine, comme l'interféron α ou le TNF α, comme décrit par exemple dans WO-A-9118454, ainsi qu'une composition refermant un anticorps dirigé contre une cytokine notamment l'interféron a et un anticorps ci-dessus dirigé contre une protéine de régulation.

La présente demande décrit un procédé d'immunisation active caractérisé en ce que l'on utilise à titre d'immunogène un composé immunogène tel que défini ci-dessus associé à un adjuvant d'immunité minéral, huileux ou de synthèse, ou encore un composé immunogène tel que défini ci-dessus couplé ou associé à une protéine augmentant son immunogénicité.

Ces immunisations peuvent être réalisées tant à titre curatif qu'à titre préventif.

A titre d'immunogène pour tous les procédés ci-dessus et ci-après, on utilise de préférence un dérivé de la protéine Tat.

La présente demande décrit un procédé d'hyperimmunisation de sujets séronégatifs ou séropositifs VIH-1, VIH-2, caractérisé en ce que l'on utilise un immunogène tel que défini ci-dessus, pour la production de sérums humains hyperimmuns, lesquels peuvent être particulièrement destinés à la sérothérapie passive par administration d'anticorps spécifiques purifiés ou de leurs fragments F(ab')2 ou Fab.

La présente demande décrit une composition pharmaceutique comprenant, à titre de principe actif curatif ou préventif, au moins un anticorps anti-protéine de régulation d'un virus tel que défini ci-dessus ou obtenu selon les procédés ci-dessus.

L'invention a enfin pour objet l'utilisation d'un composé immunogène ci-dessus pour la préparation d'un médicament destiné au traitement des effets nocifs d'une protéine de régulation d'un virus VIH-1, VIH-2.

En résumé et notamment, la présente demande décrit l'usage à titre préventif, ou curatif chez le sujet séropositif ou atteint d'ARC/Sida d'anticorps spécifiques de manière à bloquer l'action nocive de la protéine de régulation Tat d'un virus VIH-1, VIH-2. Ces anticorps spécifiques pourront provenir: 1. du sujet lui-même, induits par une immunisation active (vaccination) avec du Tat biologiquement inactivé mais immunogène, appelé Tat-toxoïde par analogie aux toxoïdes bactériens ou 2. d'un organisme étranger, allo- ou xénogénique, administré au sujet par immunisation passive (sérothérapie). Les anticorps allogéniques (chez l'homme) pourront être engendrés chez des sujets non infectés volontaires après immunisation active (vaccination) avec une protéine de régulation telle que le Tat ou ses dérivés (Tat-toxoïde ou fragments peptidiques du Tat selon l'invention). Ces anticorps administrés passivement, qu'ils soient allogéniques (humains) ou xénogéniques (animaux), pourront être des anticorps monoclonaux ou polyclonaux complets ou des fragments F(ab')2 ou Fab de l'anticorps.

Par Tat-toxoïde, comme on l'a indiqué ci-dessus on entend un peptide ou une protéine Tat traitée par un agent chimique. Ce traitement a fait perdre à la molécule les propriétés biologiques toxiques du Tat circulant (immunosuppression des cellules T; induction de la production d'interféron a par les cellules productrices d'interféron ; néoangiogenèse des cellules endothéliales; blocage de l'effet antiviral de l'interféron sur les macrophages) mais ont préservé les propriétés susceptibles d'induire la formation des anticorps, lorsque présentée et préparée de manière appropriée, couplée ou non à un "carrier", agrégée ou non, en présence ou non d'adjuvant.

On a élargi la notion de Tat-toxoïde aux fragments peptidiques immunogènes de Tat c'est-à-dire une séquence peptidique du Tat susceptible d'induire la formation des anticorps anti-Tat lorsqu'elle est présentée de manière appropriée, couplée à un carrier ou non, en présence ou non d'adjuvant.

L'invention a également pour objet des compositions pharmaceutiques.
a) Une composition pharmaceutique comprenant à titre d'agent préventif ou curatif un toxoïde viral ou un fragment ou analogue de protéine de régulation Tat selon l'invention.
b) Une composition pharmaceutique comprenant à titre d'agent préventif ou curatif des anticorps anti-Tat produits à partir d'organismes immunisés contre ladite protéine ou ses fragments F(ab')2 ou Fab, selon l'invention.

En outre la demande décrit également un kit comprenant une composition pharmaceutique vaccinale qui en plus du principe actif (Tat-toxoïde ou ses dérivés ou anticorps anti-Tat) peut comprendre un adjuvant et/ou un autre immunogène à propriétés anti-rétrovirus.

Enfin la demande décrit une composition pharmaceutique sous une forme galénique conventionnelle. En particulier on associe le principe actif selon l'invention en quantité suffisante pour être efficace d'un point de vue thérapeutique avec un diluant ou un support acceptable d'un point de vue pharmaceutique.

### EXPERIENCE 1 : Effets pathogènes de la protéine Tat circulante

### Immunosuppression des cellules T en présence de Tat:

L'effet du Tat sur des lymphocytes T activés du sang périphérique a été recherché. Des cellules mononuclées (PMBC) et des lymphocytes T (HLA 35 DR-) isolés par immunomagnétisme ont été activés par des anticorps anti-CD₃ en présence ou en absence de la molécule Tat. Après 5 jours de culture, la prolifération cellulaire a été mesurée par incorporation de thymidine H₃. Les résultats ont montré que le Tat inhibait la prolifération des cellules T HLA DR-(85% d'inhibition à la concentration de 1,5 µg par ml). Cette inhibition disparaît en présence d'anticorps spécifique anti-Tat (Intracel, U.K.).

### EXPERIENCE 2 : Effets pathogènes de la protéine Tat circulante :

### Inhibition de l'effet de l'interféron sur les macrophages en présence de Tat :

L'effet du Tat sur l'action antivirale de l'interféron α a été mesuré par le test biologique conventionnel en utilisant la mesure du pouvoir cytopathogène du virus VSV sur les cellules MDBK.

### Effet du Tat sur l'interféron exogène

Des doses croissantes de Tat ont permis d'inhiber dès de faibles concentrations suivant une courbe dose-effet pour des concentrations de Tat allant de 1 à 20 µg/ml, le pouvoir anti-viral de l'interféron exogène. Dans une expérience représentative, l'effet protecteur de l'interféron α apparent jusqu'à la dilution 1/4800 (correspondant à 150 U.I.) est réduit à la dilution 1/300 pour des concentrations de Tat de 10 µg/ml. Ainsi à la concentration de 10 µg/ml de Tat, à la dilution 4 800 de l'interféron exogène le titre de VSV est passé de 10^{3,8} (échantillons interféron sans Tat) à 10^{5,5} (échantillons interféron en présence de Tat). Dans ces expériences, l'inhibition de l'effet anti-viral de l'interféron α par la molécule Tat est supprimée en présence d'anticorps spécifiques anti-Tat (Intracel, U.K.), de même que par les anticorps de cheval que nous avons préparé (voir exemples ci-après).

### EXPERIENCE 3 : Mise en évidence chez les sujets infectes par le VIH-1 de la protéine Tat dans sa configuration extracellulaire.

Présence d'anticorps anti-Tat dans le sérum de patients séropositifs:
a) Une étude par Elisa des anticorps anti-Tat sériques utilisant comme antigène la molécule Tat native a été réalisée chez 50 sujets séropositifs et 15 sujets séronégatifs. Tous les sérums des sujets séronégatifs et 20 sérums de séropositifs n'ont pas révélé la présence d'anticorps anti-Tat avec une densité optique inférieure au seuil (cut off) (D.O. = 0,250). Parmi les 30 sérums de sujets infectés par le VIH, qui ont dépassé le seuil, 6 ont une DO supérieure à 0,500. Aucune corrélation apparente n'a pu être faite entre la présence d'anticorps anti-Tat d'une part, l'état clinique et le nombre de cellules CD4 par mm³ de sang d'autre part.
b) Une étude par Elisa d'anticorps anti-Tat sériques utilisant comme antigènes différents peptides du Tat a été réalisée chez des sujets séropositifs à différents stades de l'évolution de leur infection VIH - sujets asymptomatiques ; patients présentant des manifestations cliniques pré-Sida (ARC) et patients atteints de Sida. Les résultats de cette étude sont les suivants:
1) Les séquences peptidiques du Tat
MEPVDPRLEPWKHPG (résidus 1 - 15 en N terminal)
HQVSLSKQPTSQPRGD (résidus 65 - 80 en C terminal)
ont été reconnues par la grande majorité des sérums de séropositifs et par aucun sérum de séronégatifs (contrôles). Les réactions ont été fortement positives mais aucune corrélation n'a pu être montrée en rapport avec l'évolution de l'infection VIH et le nombre de CD4 des sujets.
2) Les autres séquences étudiées n'ont pas été reconnues de manière notable par les sérums qu'ils soient d'individus séropositifs ou D contrôles (voir Tableau 1). Seuls quelques rares sérums pour chaque séquence ont montré une densité optique supérieure au seuil (2 fois la moyenne des sérums contrôles).

**TABLEAU 1**

| | **Sujets infectés*** | | **Sujets contrôles** | |
|---|---|---|---|---|
| | **(72 sérums)** | | **( 10 sérums)** | |
| **Résidus** | **Sérums** | **D.O.** | **Sérums** | **D.O.** |
| **peptidiques du Tat** | **positifs**** | **moyenne** | **positifs**** | **moyenne** |
| 1 - 15 | 67 | 0,43 | 0 | 0,08 |
| 9 - 20 | 0 | 0,26 | 0 | |
| 22 - 37 | 7 | 0,78 | 0 | 0,41 |
| 36 - 50 | 5 | 0,86 | 0 | 0,65 |
| 46 - 60 | 6 | 0,53 | 0 | 0,40 |
| 52 - 60 | 2 | 0,32 | 0 | 0,38 |
| 56 - 70 | 0 | 0,19 | 0 | 0,20 |
| 65 - 80 | 70 | 1,01 | 0 | 0,08 |

| | | | | |
|---|---|---|---|---|
| * Les sujets infectés par le VIH sont à des stades variés; sujets asymptomatiques, patients atteints d'ARC et patients atteints de Sida. ** La réaction est considérée positive (seuil) lorsque la densité optique est 2 fois supérieure à la moyenne des séronégatifs. | | | | |

3) Il est intéressant de noter que la séquence la plus réactive (AA 65 - 80) est celle contenant les résidus RGD reconnus par les intégrines.

### EXEMPLE 1 : Préparation de la protéine Tat immunogène (Tat-toxoïde).

### Inactivation de la protéine Tat en présence de formaldéhyde

A une solution de Tat (1 mg/ml) dans du phosphate disodique 70 mM, pH 8,0, on ajoute du formaldéhyde à la concentration finale 33 mM.

Le mélange est incubé à 37 °C pendant 1,3,5,7 et 9 jours. A la fin de chacune des périodes d'incubation, un échantillon est prélevé et la réaction avec le formaldéhyde bloquée par addition de glycine à la concentration finale 100 mM.

Chaque prélèvement est dialysé pendant 1 nuit à 4°C contre 100 fois son volume de PBS (tampon phosphate salin).

### EXEMPLE 2 : Préparation de la protéine Tat immunogène (Tat-toxoïde)

### Inactivation de la protéine Tat en présence de glutaraldéhyde

A une solution de Tat (1 mg/ml) dans du phosphate disodique 70 mM, pH 8,2 on ajoute du glutaraldéhyde à la concentration finale, soit 0,026 M, soit 0,0026 M. On laisse la réaction avec le glutaraldéhyde se poursuivre pendant des temps divers variant de 1 mn à 3 h. Après le temps de réaction choisi, à la température du laboratoire, la réaction est bloquée par addition de glycine à la concentration finale de 100 mM. Les différents prélèvements sont dialysés, pendant 16 heures, à 4°C, contre 100 fois leur volume de PBS.

### EXEMPLE 3 : Préparation de la protéine Tat immunogène (Tat-toxoïde)

### Inactivation et couplage simultané à la toxine tétanique du Tat

A un mélange de toxine tétanique et de Tat dans un rapport molaire de 1 à 15, dans un tampon phosphate 70 mM- 100 mM (pH variant de 6,8 à 8,2) on a ajouté du glutaraldéhyde à la concentration finale (variant de 0,0026 M à 0,026 M) et on laisse la réaction d'inactivation et de couplage s'effectuer pendant des temps variables (3-15 mn) à la température du laboratoire. La réaction est bloquée par addition de glycine à la concentration finale 100 mM et les différents prélèvements sont dialysés pendant 16 heures, à 4°C, contre 100 fois leur volume de PBS.

### EXEMPLE 4 : Pouvoir immunogène et antigénicité des Tat-toxoïdes

### Tat-toxoïde immunogène chez la souris: Anticorps par Elisa

Le pouvoir immunogène des différentes préparations de Tat inactivé, soit par le formaldéhyde (Tat-toxoïde), soit par le glutaraldéhyde (Tat-polan), soit après couplage à la toxine tétanique (conjugué Tat-toxine tétanique), c'est à dire des composés immunogènes des exemples 1 à 3, a été déterminé chez la souris.

Des souris Swiss de 18-20g ont été immunisées par 2 injections sous-cutanées de l'immunogène des exemples 1, 2 et 3 en présence d'adjuvant complet de Freund, la seconde pratiquée 3 semaines après la 1 ère avec 20 µg de préparation en émulsion en présence d'adjuvant incomplet de Freund.

15 jours après l'injection de rappel, des prélèvements sanguins ont été pratiqués par voie intracardiaque. Les anticorps anti-Tat ont été déterminés dans les sérums par Elisa. La densité optique a été mesurée à 490 nm.

Les résultats obtenus, résumés dans le Tableau 2, montrent que toutes les préparations de Tat sont immunogènes à des degrés divers, hormis le conjugué Tat-TT traité pendant un temps court (3 minutes) par la glutaraldéhyde, qui s'est avéré toxique et a tué les souris en 24 heures (TT trop faiblement inactivé). Il est à signaler que les sérums des souris non-immunisées répondent en dessous de 0,200 (D.O) au Tat natif comme au Tat-toxoïde. De plus ces résultats montrent que le Tat natif est reconnu par les anticorps de la même façon que le toxoïde, ce qui confirme leur antigénicité équivalente et justifie encore l'utilisation du Tat-toxoïde pour immuniser.

**TABLEAU 2**

| **Préparations** | **Taux d'anticorps anti-Tat natif Densité Optique (D.O)** | **Taux d'anticorps anti-Tat toxoïde (3 jours) Densité Optique (D.O)** |
|---|---|---|
| Tat-toxoïde (1 jours) | 0,320 | 0,331 |
| Tat-toxoïde (3 jours) | 0,465 | 0,494 |
| Tat-toxoïde (5 jours) | 0,998 | 0,901 |
| Tat-toxoïde (9 jours) | 0,807 | 0,780 |
| Tat -polan (1 mn;) 0,026 M | 0,718 | 0,706 |
| Tat-polan (15 mn;) 0,026 M | 1,564 | 1,452 |
| Tat -polan (60 mn;) 0,0026 M | 1,065 | 1,113 |
| Tat -polan (3h;) 0,0026 M | 0,194 | 0,210 |
| conjugué Tat - TT (3 mn; 0,0026M) | toxique | toxique |
| conjugué Tat - TT (15 mn; 0,0026M) | 1,987 | 1,897 |
| conjugué Tat - TT (6 mn; 0,026M) | 0,824 | 0,795 |
| conjugué Tat - TT (15 mn; 0,026M) | 1,642 | 1,556 |

### EXEMPLE 5 : Toxicité aiguë

La toxicité des préparations de Tat-toxoïde et de Tat-polan a été déterminée par injection sous-cutanée à la souris Swiss de 18-20g. Les préparations de Tat-toxoïde (7 jours) et Tat-polan (15 mn; 0,026 M) ont été administrées à la dose de 100 µg par souris. Les animaux ont été observés pendant 7 jours.

Aucun signe manifeste de toxicité n'a été observé. Le poids des animaux a continué de croître. L'examen macroscopique des organes à l'autopsie n'a pas permis de révéler d'anomalies.

### EXEMPLE 6 : Anticorps anti-Tat

On a préparé des anticorps anti-Tat comme suit : on a isolé la fraction IgG sur une colonne de protéine G à partir de sérums de souris immunisées avec du Tat-polan. Les fragments F(ab')2 ont été également préparés à partir de la fraction IgG isolée par digestion pepsique. La réactivité immunologique de ces fractions a été vérifiée par Elisa.

### EXEMPLE 7 : Inactivité biologique du Tat-toxoïde

Les différents Tat-toxoïdes inactivés 1, 3, 5 , et 9 jours de l'exemple 1 sont testés dans un test d'activité d'expression du gène reporter Chloramphénicol-Acétyl-Transférase (CAT) dépendants du "Long Terminal Repeat" (LTR) du VIH-1.

Le principe de cet essai est le suivant : des cellules de lignée Hela contenant le gène CAT sous contrôle du LTR du VIH, sont mises en contact avec le Tat natif ou les toxoïdes pendant 6h. Après 24h de culture, les cellules sont lysées et la quantité de CAT produite est mesurée par un test d'activité du CAT qui mesure le pourcentage d'acétyl-CoA accroché au chloramphénicol. Si la molécule Tat est active, il y aura un fort pourcentage de chloramphénicol acétylé, sinon ce pourcentage sera faible. La culture de ces cellules adhérentes se fait de manière standard en milieu RPMI, 10% FCS. La concentration de Tat ajoutée pour avoir un effet-dose va de 1 µg à 10 µg par ml de surnageant.

Les résultats présentés ici montrent que le Tat-toxoïde est totalement inactivé après 3 jours, avec une activité résiduelle faible à 1 jour.

| **Préparations** | **Acétylation du chloramphénicol (%)** |
|---|---|
| Tat natif | 100 |
| Tat-toxoïde (1 jours) | 18 |
| Tat-toxoïde (3 jours) | 3 |
| Tat-toxoïde (5 jours) | 2 |
| Tat-toxoïde (9 jours) | 3 |

### EXEMPLE 8 : Absence d'effets pathogènes du Tat-toxoïde

### a) Absence d'immunosuppression des cellules T en présence de Tat-toxoïde (contrairement aux expériences 1 et 2).

L'effet du Tat-toxoïde (5 jours) sur des lymphocytes T activés du sang périphérique a été recherché. Des cellules mononuclées (PMBC) et des lymphocytes T (HLA 35 DR-) isolés par immunomagnétisme ont été activés par des anticorps anti-CD₃ en présence ou en absence de composés immunogènes de l'exemple 1. Après 5 jours de culture, la prolifération cellulaire a été mesurée par incorporation de thymidine H₃. Les résultats ont montré que, contrairement au Tat natif, le Tat-toxoïde n'inhibait pas la prolifération des cellules T HLA DR-. Ainsi, à la dose de 5 µg/ml dans la culture, en présence de Tat-toxoïde, la prolifération des cellules était identique à celle du contrôle.

### b) Absence d'inhibition de l'effet de l'interféron α sur les macrophages en présence de Tat-toxoïde:

L'effet du Tat-toxoïde sur l'action antivirale de l'interféron α a été mesuré par le test biologique conventionnel en utilisant la mesure du pouvoir cytopathogène du virus VSV sur les cellules MDBK.

### Effet du Tat-toxoïde sur l'interféron exogène

Contrairement au Tat natif, des doses équivalentes de Tat-toxoïde de l'exemple 1 n'ont pas permis d'inhiber le pouvoir anti-viral de l'interféron exogène. Dans une expérience représentative, l'effet protecteur de l'interféron a apparent jusqu'à la dilution 1/4800 (correspondant à 150 U.I.) est maintenu en présence de 50 µg de Tat-toxoïde, alors qu'il est diminué au 1/300 avec le Tat natif. A la dilution 4 800 de l'interféron exogène le titre de VSV est maintenu au niveau du contrôle à 10^{3,8} (échantillons interféron avec Tat-toxoïde) alors qu'il passe à 10^{5,5} (échantillons interféron en présence de Tat).

### EXEMPLE 9 : Rôle protecteur des anticorps anti-Tat-toxoïde face aux effets du Tat natif.

Des anticorps anti-Tat-toxoïdes de l'expérience 1 (traitement au formaldéhyde 3 jours) ont été préparés chez des chevaux hyperimmunisés. A partir de ces anticorps des fragments F(ab')2 ont été aussi préparés selon la procédure décrite dans l'exemple 6.

Les anticorps, comme les fragments F(ab')2, inhibent les différentes activités biologiques du Tat natif dans les différents tests: transactivation du LTR du VIH (essai CAT) (voir exemple 7), immunosuppression (voir expérience 1), inhibition de l'effet de l'interféron α exogène sur culture (voir expérience 2). Les expériences 1, 2, et l'exemple 7 ont été repris avec du Tat natif et en préincubant ou non ce Tat natif 1h à 37° avec les anticorps ou fragments F(ab')2, à la dose de 40 µg d'anticorps pour 1 µg de Tat natif. Les résultats ont montré que les anticorps inhibent l'action du Tat natif.

### EXEMPLE 10 : Redressement systématique de la prolifération cellulaire grâce à la combinaison anticorps anti-Tat et anticorps anti-interféron α : synergie des anticorps anti-Tat et anti-interféron α.

On a observé que des PBLs (cellules mononucléées du sang périphérique) de sujets sains infectées in vitro avec du VIH-1 et cultivées 6 jours exerçaient une activité immunosuppressive.

En effet, si l'on ajoute de telles cellules infectées depuis 6 jours, ou non infectées) irradiées en proportion de 1 pour 5 à des cellules autologues activées par la protéine Staphylococcus Enterotoxin B (SEB), on observe au bout de 4 jours que la prolifération des cellules autologues est amoindrie de 80% par rapport aux contrôles (cellules non infectées).

Dans ce modèle, si on ajoute à la culture des cellules infectées in vitro des anticorps anti-interféron α, ces cellules perdent alors leur effet suppresseur dans 50% des sujets. Chez 20% des cas, les cellules suppressives perdent 60% de leur effet suppresseur, et chez 30% cet effet suppresseur est significativement maintenu.

Si on ajoute aux anticorps anti-interféron α des anticorps anti-Tat (monoclonaux décrits en expérience 1, ou polyclonaux de cheval de l'exemple 9), c'est dans 100% des cas que les cellules infectées in vitro perdent leur effet suppresseur.

Ces expériences montrent le rôle combiné toxique de Tat et de l'interféron α dans l'immunosuppression induite par le VIH1 et qu'une i association d'anticorps anti-interféron et anti-Tat restaure une immunité normale.

Les exemples suivants ne font pas parti de l'invention.

### EXEMPLE 12 : Préparation de la protéine Nef immunogène (Nef-toxoïde)

### Inactivation de la protéine Nef en présence de formaldéhyde.

A une solution de protéine Nef (1g/ml) dans du phosphate disodique 70mM, pH 8,0, on ajoute du formaldéhyde à la concentration finale de 33 mM.

Le mélange est incubé à 37°C pendant 1, 3, 5, 7 et 9 jours. A la fin de chacune des périodes d'incubation, un échantillon est prélevé et la réaction avec le formaldéhyde bloquée par addition de glycine à la concentration finale 100mM.

Chaque prélèvement est dialysé pendant 1 nuit à 4°C contre 100 fois son volume de PBS (tampon phosphate salin), et le Nef-toxoïde isolé.

### EXEMPLE 13 : Préparation de la protéine Nef immunogène (Nef-polan)

### Inactivation de la protéine Nef en présence de glutaraldéhyde.

A une solution de Nef (1mg/mlm) dans du phosphate disodique 70mM, pH 8,2, on ajoute du glutaraldéhyde à la concentration finale, soit 0,026 M, soit 0,0026 M. On laisse la réaction avec le glutaraldéhyde se poursuivre pendant des temps divers variant de 1mn jusqu'à 3 H. Après le temps de réaction choisi, à la température du laboratoire, la réaction est bloquée par addition de glycine à la concentration finale de 100 mM. les différents prélèvements sont dialysés, pendant 16 heures, à 4°C, contre 100 fois leur volume de PBS, et le Nef-polan isolé.

### EXEMPLE 14 : Pouvoir immunogène et antigénicité des Nef-toxoïdes

### Nef-toxoïde immunogène chez la souris : Anticorps par Elisa.

Le pouvoir immunogène des différentes préparations de Nef inactivé, soit par le formaldéhyde (Nef-toxoïde), soit par le glutaraldéhyde (Nef-polan), c'est à dire les composés immunogènes des exemples 12 et 13, a été déterminé chez la souris.

Des souris Swiss de 18-20 g on été immunisées par 2 injections sous-cutanées de l'immunogène des exemples 12 et 13 en présence d'adjuvant de Freund, la seconde pratiquée 3 semaines après la première, avec 20 µg de préparation en émulsion en présence d'adjuvant incomplet de Freund.

15 jours après l'injection de rappel, les prélèvements sanguins ont été pratiqués par voie intracardiaque. Les anticorps anti-Nef ont été déterminés dans les sérums par Elisa. La densité optique a été mesurée à 490 nm.

Les résultats obtenus, résumés dans le tableau ci-après, montrent que toutes les préparations de Nef sont immunogènes à des divers degrés. Il faut signaler que les sérums de souris non immunisées donnent un niveau de réponse inférieur à 0,2 (en D.O) dans ces essais Elisa.

Ces résultats montrent que la protéine Nef native est reconnue par les anticorps de la même façon que le toxoïde, ce qui confirme leur antigénicité équivalente et justifie encore l'utilisation du Nef-toxoïde pour immuniser.

| **Préparations** | **Taux d'anticorps anti-Nef natif Densité Optique (D.O)** | **Taux d'anticorps anti-Nef toxoïde (3 jours) Densité Optique (D.O)** |
|---|---|---|
| Nef-toxoïde (1 jours) | 0,56 | 0,5 |
| Nef-toxoïde (3 jours) | 0,78 | 0,8 |
| Nef-toxoïde (5 jours) | 0,99 | 1,01 |
| Nef-toxoïde (9 jours) | 0,65 | 0,75 |
| Nef -polan (1 mn;) 0,026 M | 1,12 | 0,98 |
| Nef -polan (15 mn;) 0,026 M | 1,69 | 1,56 |
| Nef -polan (60 mn;) 0,0026 M | 1,31 | 1,41 |
| Nef -polan (3h;) 0,0026 M | 0,35 | 0,41 |

## Revendications

1. Une composition pharmaceutique comprenant un composé immunogène administrable à l'homme car dénué de toxicité, **caractérisé en ce que** ledit composé immunogène dérive d'une protéine de régulation d'un virus VIH-1 ou VIH-2, ou d'un de ses fragments peptidiques, par traitement à l'aide d'un agent chimique ou par couplage avec une protéine porteuse activée par un pré-traitement à l'aide d'un aldéhyde, lui permettant d'être reconnu par des anticorps dirigés contre ladite protéine de régulation native, et de conserver des propriétés immunogènes suffisantes pour créer des anticorps neutralisant ou bloquant ladite protéine native, tout en ayant perdu au moins 50 % des propriétés biologiques toxiques de ladite protéine native, ladite protéine de régulation étant la protéine Tat.

2. Un composé immunogène tel que défini à la revendication 1 pour son utilisation dans une méthode de traitement du corps humain ou animal.

3. Un composé immunogène selon la revendication 2, **caractérisé en ce qu'**il dérive de la protéine Tat, par traitement à l'aide d'un agent chimique.

4. Un composé immunogène selon la revendication 3, **caractérisé en ce que** le traitement à l'aide d'un agent chimique est un traitement à l'aide d'un aldéhyde.

5. Une composition pharmaceutique **caractérisée en ce qu'**elle comprend un composé immunogène tel que défini à l'une des revendications 1 à 4, associé à une protéine Gag, Pol ou Env d'un virus VIH-1, VIH-2, HTLV-1 ou HTLV-2.

6. Une composition pharmaceutique **caractérisée en ce qu'**elle comprend un composé immunogène tel que défini à l'une des revendications 1 à 4, associé à une protéine gp120/gp160, native ou inactivée par traitement physique, chimique, génétique ou immunologique ou à une fraction peptidique de cette protéine, modifiée ou non.

7. Une composition vaccinale renfermant :
- un composé immunogène tel que défini à l'une des revendications 1 à 4, et
- un composé immunogène qui est une cytokine sous une forme différente de sa forme native et inactivée ou un analogue inactif ou un fragment inactif ou inactivé de cytokine.

8. Un vaccin renfermant, à titre d'immunogène, un composé tel que défini à l'une des revendications 1 à 4, associé à un adjuvant d'immunité minéral, huileux ou de synthèse, ou à une protéine augmentant son immunogénicité.

9. Utilisation d'un composé immunogène tel que défini à l'une des revendications 1 à 4, pour la préparation d'un médicament curatif ou préventif destiné au traitement ou à la prévention des effets nocifs des protéines de régulation d'un virus VIH-1 ou VIH-2.

## Claims

1. Pharmaceutical composition comprising an immunogenic compound which can be administered to humans, since it is non-toxic, **characterized in that** said immunogenic compound is derived from an HIV-1 or HIV-2 virus regulatory protein or one of its peptide fragments by chemical treatment or by coupling with a carrier protein activated by pre treatment with the aid of an aldehyde, which enables it to be recognized by antibodies to the said native regulatory protein, and to retain sufficient immunogenic properties to create antibodies which neutralize or block said native protein, while having lost at least 50% of toxic biological properties of said native protein, said regulatory protein being the Tat protein.

2. Immunogenic compound as defined in claim 1 for use in a method for treating the human or animal body.

3. Immunogenic compound according to claim 2 **characterized in that** it is derived from the protein Tat by chemical treatment.

4. Immunogenic compound according to claim 3, **characterized in that** the chemical treatment is a treatment with the aid of an aldehyde.

5. Pharmaceutical composition, **characterized in that** it comprises an immunogenic compound as defined in one of claims 1 to 4, combined with a Gag, Pol or Env protein of an HIV-1, HIV-2, HTLV-1 or HTLV-2 virus.

6. Pharmaceutical composition, **characterized in that** it comprises an immunogenic compound as defined in one of claims1 to 4, combined with a gp120/gp160 protein, which is native or inactivated by physical, chemical, genetic or immunological treatment, or with a modified or non-modified peptide fraction of this protein.

7. A vaccine composition comprising
- an immunogenic compound as defined in one of claims 1 to 4, and
- an immunogenic compound which is a cytokine in a form different to its native form and is inactivated or an inactive analogue or an inactive or inactivated fragment of the cytokine.

8. A vaccine comprising a compound as defined in one of claims 1 to 4 as immunogenic agent, combined with a mineral, oily or synthetic immunity adjuvant or to a protein which increases its immunogenicity.

9. Use of an immunogenic compound as defined in one of claims 1 to 4 for the preparation of a curative or preventative medicament intended for the treatment or prevention of the harmful effects of HIV-1, HIV-2 virus regulatory proteins.

## Patentansprüche

1. Pharmazeutische Zusammensetzung, umfassend eine dem Menschen verabreichbare detoxifizierte immunogene Verbindung, **dadurch gekennzeichnet, dass** die immunogene Verbindung von einem Regulationsprotein eines 1-HIV- oder 2-HIV-Virus oder von einem seiner Peptidfragmente durch chemische Behandlung oder durch Kopplung mit einem Trägerprotein, aktiviert durch eine Vorbehandlung mit einem Aldehyd, abgeleitet ist, was die Wiedererkennung durch Antikörper, die gegen das native Regulationsprotein gerichtet sind und die Aufrechterhaltung von ausreichenden immunogenen Eigenschaften ermöglicht, um Antikörper zu erzeugen, die das native Protein neutralisieren oder blockieren, während mindestens 50% der toxischen biologischen Eigenschaften des nativen Proteins verloren gehen, wobei das Regulationsprotein das Tat-Protein ist.

2. Immunogene Verbindung, definiert nach Anspruch 1, zur Verwendung in einem Behandlungsverfahren für den menschlichen oder tierischen Körper.

3. Immunogene Verbindung nach Anspruch 2, **dadurch gekennzeichnet, dass** sie von dem Tat-Protein durch chemische Behandlung abgeleitet ist.

4. Immunogene Verbindung nach Anspruch 3, **dadurch gekennzeichnet, dass** die chemische Behandlung eine Behandlung mit einem Aldehyd ist.

5. Pharmazeutische Zusammensetzung, **dadurch gekennzeichnet, dass** sie eine immunogene Verbindung, definiert nach einem der Ansprüche 1 bis 4, umfaßt, die an ein Protein Gag, Pol oder Env eines 1-HIV-, 2-HIV-, 1-HTLV-oder 2-HTLV-Virus assoziiert ist.

6. Pharmazeutische Zusammensetzung, **dadurch gekennzeichnet, dass** sie eine immunogene Verbindung, definiert nach einem der Ansprüche 1 bis 4, umfaßt, die an ein Protein gp120/gp160, das nativ oder durch physikalische, chemische, genetische oder immunologische Behandlung inaktiviert ist, oder an eine Peptidfraktion dieses Proteins, modifiziert oder nicht, assoziiert ist.

7. Vakzinsusammensetzung, umfassend
- eine immunogene Verbindung, definiert nach einem der Ansprüche 1 bis 4 und
- eine immunogene Verbindung, die ein Cytokin ist, das in einer von seiner nativen Form unterschiedlichen Form vorliegt und inaktiviert ist oder ein inaktives Analogon oder ein inaktives oder inaktiviertes Fragment des Cytokins.

8. Vakzin, umfassend als Immunogen eine Verbindung, definiert nach einem der Ansprüche 1 bis 4, die an ein mineralisches, ölhaltiges oder synthetisches Immunitätsadjuvants oder ein Protein, das die Immunogenität erhöht, assoziiert ist.

9. Verwendung einer immunogenen Verbindung, definiert nach einem der Ansprüche 1 bis 4 zur Herstellung eines heilenden oder vorbeugenden Arzneimittels, bestimmt für die Behandlung oder die Vorbeugung schädlicher Wirkungen von Regulationsproteinen der 1-HIV-, 2-HIV-Viren.


### LISTE DE SEQUENCES

### (1) INFORMATION GENERALE :

(i) DEPOSANT :
   (A) NOM : NEOVACS
   (B) RUE : 117, rue Vieille du Temple
   (C ) VILLE : PARIS
   (E) PAYS : FRANCE
   (F) CODE POSTAL : 75003
(ii) TITRE DE L'INVENTION : Nouveaux immunogènes, de nouveaux anticorps, leur procédé de préparation et des compositions pharmaceutiques les renfermant.
(iii) NOMBRE DE SEQUENCES : 1
(iv) FORME LISIBLE PAR ORDINATEUR :
   (A) TYPE DE SUPPORT : Floppy disk
   (B) ORDINATEUR : IBM PC compatible
   (C ) SYSTEME D'EXPLOITATION : PC-DOS/MS-DOS
   (D) LOGICIEL : Patentln Release # 1.0, Version # 1.25 (OEB)

### (2) INFORMATION POUR LA SEQUENCE ID NO : 1 :

(i) CARACTERISTIQUES DE LA SEQUENCE :
   (A) LONGUEUR : 16 acides aminés
   (B) TYPE : acide aminé
   (C ) NOMBRE DE BRINS : simple
   (D) CONFIGURATION : linéaire
(ii) TYPE DE MOLECULE : peptide
(xi) DESCRIPTION DE LA SEQUENCE : SEQ ID NO : 1 :
   His Gln Val Ser Leu Ser Lys Gln Pro Thr Ser Gln Pro Arg Gly Asp
   1 5 10 15